Europäisches Patentamt

European Patent Office

Office européen des brevets

(19)

(11) Publication number: **0 161 816**
**A2**

(12)

# EUROPEAN PATENT APPLICATION

(21) Application number: **85302653.2**

(51) Int. Cl.⁴: **A 61 K 37/24,** A 61 K 35/22

(22) Date of filing: **16.04.85**

(30) Priority: **17.04.84  GB 8409961**

(43) Date of publication of application: **21.11.85**
**Bulletin 85/47**

(84) Designated Contracting States: **AT BE CH DE FR GB IT LI LU NL SE**

(71) Applicant: **IMPERIAL CHEMICAL INDUSTRIES PLC, Imperial Chemical House Millbank, London SW1P 3JF (GB)**
Applicant: **G D SEARLE & CO LTD, PO Box 53 Lane End Road, High Wycombe Bucks HP12 4HL (GB)**

(72) Inventor: **Gregory, Harold, Mereside Alderley Park, Macclesfield Cheshire SK10 4TG (GB)**
Inventor: **McCullagh, Keith Graham, Windrush House 30 Manor Park, Princes Risborough Buckinghamshire (GB)**

(74) Representative: **Mack, John Richard et al, Imperial Chemical Industries PLC Legal Department: Patents PO Box 6 Bessemer Road, Welwyn Garden City Hertfordshire AL7 1HD (GB)**

(54) Urogastrone.

(57) Pharmaceutical compositions comprise human urogastrone or a urogastrone fragment in combination with a known anti-ulcer agent as active ingredient and such active ingredients may be used for the manufacture of a medicament for promoting healing of gastrointestinal ulcers.

EP 0 161 816 A2

0161816

# UROGASTRONE

The present invention relates to human urogastrone and related polypeptides and in particular to their use in combination with a known anti-ulcer agent in the treatment of ulcers of the gastrointestinal tract and in the manufacture of medicaments therefor. The present invention also relates to pharmaceutical compositions comprising human urogastrone or a related polypeptide and a known anti-ulcer agent as active ingredients. The human urogastrone is preferably recombinant derived beta-urogastrone produced by microbial methods.

Urogastrone is a polypeptide hormone (protein) synthesized in the salivary glands and duodenal Brunner's glands of normal humans (see for example Heitz et al, Gut, 19: 408, 1978). Urogastrone is known (see for example Elder et al, Gut 16, 887, 1975 and Koffman et al, Gut 23: 951, 1982) to inhibit acid secretion in the stomach of humans when administered by a systemic route.

Urogastrone was first identified and described in 1939 (by Gray et al, Science, 89: 489, 1939) as a component in human urine which inhibits gastric (stomach) acid secretion in experimental animals when given parenterally. This component, named "urogastrone", was completely sequenced and its structure published in 1975 (Gregory, Nature (Lond.) 247: 325, 1975). Urogastrone is probably synthesized in man as part of a larger "pro-peptide" molecule from which it is cleaved by specific proteases to liberate the active form of the protein (Frey et al, Proc. Nat. Acad. Sci, 76: 6294, 1979). This active form of urogastrone, composed of 53 amino acids is known as beta-urogastrone. Further degradative cleavage in the body yields a slightly smaller form of the protein of 52 amino acids known as gamma-urogastrone which is also found in human urine and which differs only from beta-urogastrone in lacking the carboxy-terminal arginine residue (see for example, Gregory and Preston, Int. J. Peptide Prot. Res. 9: 107, 1977). Both polypeptides have equal activity in inhibiting acid secretion when administered intravenously to animals (see for example UK Patent No. 1,394,846). Other urogastrone species produced by further enzymatic cleavage having for example only amino acids 1-47 and 1-46 are also known to inhibit acid secretion when administered by intravenous or subcutaneous routes (see for example British Patents Nos. 1,461,105 and 1,461,106).

A related polypeptide called epidermal growth factor (mEGF) was isolated and characterized from mouse salivary glands (Cohen, J. Biol. Chem., 237: 1555, 1962). Mouse EGF consists of a polypeptide of 53 amino acids which resembles beta-urogastrone but differs in detailed amino acid sequence. Mouse EGF is known to have similar biological activities to urogatrone in inhibiting gastric acid secretion and is also known to stimulate the proliferation of epithelial tissues (see for example UK Patent No. 1,417,776 and Carpenter and Cohen, Ann. Rev. Biochem. 48: 193, 1979).

In addition to their antisecretory properties urogastrone and mouse EGF are known to protect the gastrointestinal mucosa of animals against damaging stimuli and ulcer formation when administered concurrently with or prior to the injurious stimuli (see for example Kirkegaard et al, Gastroenterology 85: 1277, 1983 and Konturek et al, Gut 22: 927, 1981). This "cytoprotective" effect is known to occur when the polypeptides are administered parenterally at doses

0161816

below that necessary to cause inhibition of acid secretion or when the polypeptides are administered directly into the gastrointestinal lumen from which absorption into the blood stream does not occur and from which acid secretion is not inhibited (see for Example Kirkegaard et al, Gastroenterology 85: 1277, 1983 cited above).

The present invention relates to the discovery that urogastrone will promote the healing of ulcers of the mucosa and submucosa of the gastrointestinal tract when administered orally or into the gastrointestinal lumen after the ulcer has formed. This effect is truly surprising since it has been found that urogastrone at high doses infused into the stomach lumen has no effect on gastric acid secretion or on the rate of gastrointestinal cell proliferation and growth and, since, of course, cytoprotection is unable to account for an increased rate of healing after the ulcer has formed.

The present invention is also based on the discovery that the use of urogastrone or a urogastrone fragment in combination with a known anti-ulcer agent results in enhanced healing of ulcers of the gastrointestinal tract, over and above that obtained by use of the known anti-ulcer therapy alone.

According to the present invention there is provided the use of (1) human urogastrone or a urogastrone fragment and (2) a known anti-ulcer agent for the manufacture of a medicament for promoting the healing in mammals of gastrointestinal ulcers. The medicament is preferably for promoting the healing of duodenal ulcers and is most preferably in an orally administrable form. The medicament is preferably associated with written or printed instructions for its use. The instructions will, for example, state the dosage(s), the most advantageous dose regime and may state the conditions in which use of the medicament would be contraindicated.

According to a further feature of the present invention there is provided a method of treating gastrointestinal ulcers of a mammal which comprises the administration of (1) human urogastrone or a urogastrone fragment and (2) a known anti-ulcer agent to said mammal. The method of the present invention is of particular interest in promoting duodenal ulcer healing and the urogastrone or urogastrone fragment and known anti-ulcer agents are preferably administered orally.

According to a further feature of the present invention there is provided a pharmaceutical composition which comprises (1) human urogastrone or a urogastrone fragment and (2) a known anti-ulcer agent as active ingredients in association with a pharmaceutical carrier or excipient. The pharmaceutical composition is preferably in a form suitable for oral administration.

According to a further feature of the present invention there is provided for use in medicine, especially for use in promoting the healing of gastrointestinal ulcers such as duodenal ulcers in mammals, a pharmaceutical composition, preferably adapted for oral administration, which comprises (1) human urogastrone or a urogastrone fragment and (2) a known anti-ulcer agent as active ingredients.

According to a further feature of the present invention there is provided an agent for promoting the healing of gastrointestinal ulcers in mammals which comprises as active ingredient (1) human urogastrone or a urogastrone fragment and (2) a known anti-ulcer agent. The agent for promoting healing is preferably in a form adapted for oral administration and is most preferably, a duodenal ulcer healing agent.

Urogastrone per se having the amino acid sequence and structure defined by Gregory H, in Nature (Lond) 247: 325, 1975 is preferably used. If desired however a urogastrone fragment capable of promoting the healing of gastrointestinal ulcers may be used.  Such a fragment may for example be a polypeptide comprising only amino acids 1-46, 1-47, 1-48, 1-49, 1-50, 1-51 or 1-52.  It will be appreciated that references herein to urogastrone or a urogastrone fragment include such polypeptides which carry methionine or formylmethionine at their N-termini as well as such polypeptides which carry a peptide sequence of up to 12 amino acid residues at their N-termini, which sequence may in turn be preceded by methionine or formylmethionine, providing that the total number of amino acid residues in the sequence of amino acid residues preceding the N-terminus does not exceed 12.

Human urogastrone or a urogastrone fragment may if desired be used as the sole growth factor or may be used in combination with other growth factors such as (1) those transforming growth factors which compete with human urogastrone for receptor binding and which do not require human urogastrone for the induction of colony formation in soft agar; (2) those transforming growth factors which do not compete with urogastrone for receptor binding and which do not require urogastrone for colony formation in soft agar; and (3) those transforming growth factors which both compete for urogastrone receptors and require urogastrone for colony formation in soft agar.

Urogastrone and mEGF are present in mammalian tissues and fluids in amounts too small to provide a source of the proteins for human clinical use and the polypeptides are too large to be readily produced by conventional peptide chemical synthesis.  However, beta-urogastrone is known to be capable of production from a synthetic oligonucleotide gene expressed in microbial cells and the expressed protein can thence be recovered (see for example European Patent Publication 40639 and also Sassenfeld and Brewer, Biotechnology 2: 76, 1984).

Urogastrone may be administered therapeutically as part of a common oral formulation which includes a known anti-ulcer agent.

Examples of such known agents: histamine H-2 receptor antagonists, e.g. cimetidine, ranitidine, famotidine, and those described in European Patent publications 60094 and 60697; gastric specific anticholinergic agents such as pirenzepine; prostaglandin E2 analogues such as misoprostol or arboprostil; agents such as sucralfate or carbenoxolone; $H^+/K^+$ ATPase inhibitors such as omeprazole; and antacids such as aluminium hydroxide/magnesium hydroxide mixtures.

Where human urogastrone or a urogastrone fragment is to be used in combination with a transforming growth factor the transforming growth factor is preferably TGF-beta, for example derived from human platelets (see PCT Application No. PCT/US83/01460).

The oral pharmaceutical composition may be formulated by means known to the art into the form of, for example, aqueous or oily solutions or suspensions, emulsions, tablets, capsules, lozenges, chewing gums or dispersible powders.

A preferred oral pharmaceutical composition is one suitable for administration in unit dosage form, for example an aqueous or oily solution or suspension or an emulsion containing between 0.01 mg. and 10 mg., preferably 0.1 to 1 mg., of urogastrone or urogastrone fragment per 5 ml., or a composition in unit dosage form, for example a tablet, capsule, lozenge or stick of chewing gum each containing between 0.01 mg. and 10 mg., preferably 0.1 to 1 mg. of urogastrone or urogastrone fragment.

The known anti-ulcer agent may be present in the composition in an amount consistent with its known therapeutic activity. Thus, for example, an oral composition containing cimetidine may contain between 100 and 1000 mg. of cimetidine.

The oral pharmaceutical composition will be administered to a mammal for the treatment of gastrointestinal ulcers so that each patient will receive an oral dose of between 0.1 and 100 µg/kg/day. A preferred dose range is between 1 and 100 µg/kg/day, and a particularly preferred range is between 1 and 10 µg/kg./day. The composition may be administered 1 to 4 times, and preferably once, per day and will preferably be administered before food, for example about an hour before food.

The oral pharmaceutical composition containing the known anti-ulcer agent may be administered in a manner consistent with the known use of such a compound. Thus, for example, a composition containing cimetidine may be administered to man such that the dose may be between 200 and 1000 mg. per day.

Where the urogastrone or urogastrone fragment and known anti-ulcer agent are to be administered parenterally each patient will advantageously receive 0.1-100 µg/kg./day, preferably 0.1 to 20 µg./kg./day, especially 1 to 10 µg./kg./day. The known anti-ulcer agent may be present in the composition for parenteral administration in an amount consistent with its known therapeutic activity. Thus for example where cimetidine is the known anti-ulcer agent to be used then preferably from 800 to 2500 mg. of cimetidine may be administered daily.

The invention is exemplified by experiments

0161816

in rats in which duodenal ulcers similar to human duodenal ulcers are induced by the subcutaneous administration of sufficient amounts of the toxic agent, cysteamine. Five to seven days after formation of the duodenal ulcers, groups of rats are treated with oral aqueous solutions of purified recombinant derived beta-urogastrone or with oral doses of a control solution containing no urogastrone or with oral doses of cimetidine, in amounts sufficient to inhibit gastric acid secretion or with identical doses of urogastrone and cimetidine as those used alone but given in combination with each other. Significantly higher percentages of healed ulcers are shown to occur in rats treated with oral beta-urogastrone or with oral cimetidine than in control rats receiving no therapy.

However, an even higher percentage of healed ulcers is shown to occur in rats receiving the combined treatment of cimetidine and beta-urogastrone than in rats receiving either urogastrone or cimetidine alone and this combination effect is shown to be statistically highly significant.

The use of a combination of beta-urogastrone with a known anti-ulcer agent is thus potentially capable of increasing both the rate and the extent of gastrointestinal ulcer healing and may thus render a wider range of patients amenable to such ulcer healing.

**Example 1**

**Enhanced Healing of duodenal ulcers following oral administration of urogastrone in combination with an antisecretory agent.**

Rats with perforating chronic duodenal ulcers which had been confirmed by laparotomy were selected from a larger number of animals given two doses of the ulcerogenic amine, cysteamine, as described in the following protocol. These animals were randomly assigned to treatment groups of 15 rats each.

The protocol utilises 2 doses of cysteamine (300 mg./kg. and 150 mg./kg., 6 hours apart). This procedure produces deep perforating duodenal ulcers in a majority of the rats injected. The "chronic" ulcers heal extremely slowly. Previous studies have shown that after 50 days only 20% have healed as judged by re-epithelialization of the surface. Even after 200 days, 36% remain unhealed.

In order to provide for a relaible experimental protocol, rats given 2 doses of cysteamine are subjected to an intra-abdominal inspection by laparotomy 5 days after induction. Only those rats in which perforation of the ulcer to the duodenal external serosal surface has occurred are included in the experimental groups. Rats with non-perforating ulcers are discarded. Therapy by drug or placebo then begins on day 7 post-induction. On termination after 50 days of therapy the duodenum is removed from all rats, fixed and stained with PAS and the numbers of healed and unhealed ulcers determined after macroscopic examination of the mucosal surface with a stereomicroscope. Healing is defined as restoration of complete mucosal integrity by re-epithelialisation of the surface. This is confirmed by subsequent histology.

In the example, therapy, which commenced on day 7 following cysteamine and continued for 50 days was as follows:

Group I    Combined treatment with Cimetidine (100 mg./ rat/day) and urogastrone (6 µg/rat/day) in drinking water.

Group II    Control - no treatment

Group III    Cimetidine alone - 100 mg./rat/day in drinking water.

Group IV    Urogastrone alone - 6 µg/rat/day in drinking water

At the termination of the experiment, the numbers of rats with healed and unhealed ulcers in each group were as follows:

| Group | Treatment | N | Healed | Unhealed | Percentage |
|-------|-----------|----|--------|----------|------------|
| I | Urogastrone and Cimetidine | 15 | 13 | 2 | 87% |
| II | None (control) | 15 | 2 | 13 | 13% |
| III | Cimetidine alone | 15 | 6 | 9 | 40% |
| IV | Urogastrone alone | 14 | 6 | 8 | 43% |

Statistical evaluation of this data was performed using Fisher's exact test. Probability values calculated for various comparisons were as follows:

Urogastrone and Cimetidine v control      $p < 0.01$

Urogastrone and Cimetidine v urogastrone      $p < 0.01$

Urogastrone v Control      $p < 0.05$

Cimetidine v Control      $p < 0.05$

This experiment shows that the combined therapy of urogastrone and cimetidine produces' healing percentages which are better than with either therapy alone and which reach high levels of statistical significance.

Further statistical analysis of the data is performed by means of an analysis of variance adapted for proportions. Three effects can be tested, each one, leading to a Chi-square statistic with 1 degree of freedom from which probability values can be determined. Results are as follows:

| Effect | Chi-square | Probability | Comment |
|---|---|---|---|
| Urogastrone alone | 8.6 | p<0.005 | Significant |
| Cimetidine alone | 7.6 | p<0.01 | Significant |
| Interaction | 0.3 | p>0.50 | Not significant |

The absence of a significant effect for interaction indicates that the effects of urogastrone and cimetidine when combined are strictly additive. This is evidence that the beneficial effect of each treatment is obtained via a different mechanism such that when combined there is an additive effect.

This experiment shows oral urogastrone to be of value in the treatment of rat duodenal ulcers and that such benefit is achieved even in the presence of an anti-secretory agent. It is concluded that combination therapy gives healing rates unobtainable by single therapy.

**Example 2**

**Oral administration of urogastrone at high doses does not inhibit acid secretion.**

In this example, oral administration of solutions of recombinant beta-urogastrone is shown to have no effect on gastric acid secretion in dogs with Heidenhain pouches whereas intravenously administered urogastrone and mEGF inhibited acid secretion.

Male beagle dogs (12-22kg) were prepared with isolated Heidenhain (vagal denervated) pouches derived from the fundic area or the stomach by the method of Rudick et al, J.Surg.Res.7: 383, 1967. Animals were allowed 4-6 weeks to recover from surgery and given a 2-3 months training period to standardise laboratory behaviour and secretory responses. The dogs were fasted for 23 hours prior to use, the pouch then flushed with warm water and histamine infused subcutaneously at a rate of 10 µg/min. Pouch secretions were collected every 15 minutes, their volume measured and aliquots titrated to pH 7.0 with 100 mM NaOH. Urogastrone solutions were administered either intravenously via a cephalic foreleg vein or orally once a secretory plateau had been attained and secretion was monitored for a further 3 hours.

Following an intravenous bolus dose of urogastrone, rapid inhibition of the acid secretion response to histamine occurred, peak inhibition being achieved within 30 minutes of urogastrone injection. Dose response studies gave log-linear dose response regression plots from which the dose required to produce a 50% inhibition of acid secretion was calculated as 0.27 µg/kg. Recombinant beta-urogastrone, administered to the same dogs as a single oral solution dose had no effect on acid secretion even when given at doses of 4 mg. (approximately 200 µg/kg.)

This experiment shows that urogastrone is unable to inhibit gastric acid secretion when administered orally, presumably because local action of urogastrone in the stomach is unable to influence acid secretion and because urogastrone is not absorbed to any extent from the small intestine. The effect of urogastrone in causing an increased rate of ulcer healing when given orally is therefore not a reflection of the known acid secretion inhibiting property of the polypeptide and is therefore surprising.

- 13 -

## Pharmaceutical Composition Example

### Example 1

An intimate mixture of urogastrone (0.5 g.), cimetidine (2 kg.), Avicel (microcrystalline cellulose) (2 kg.), magnesium stearate (20 g.) and starch (100 g.) was compressed and the compressed mixture was then broken down into granules by passge through a 16-mesh screen. The resultant granules were compressed into tablets, each of which contains 50 µg. of urogastrone.

For intestinal lesions in the ileum and colon the tablets are enteric coated with cellulose acetate phthalate.

### Example 2

A clear syrup comprising urogastrone and cimetidine as active ingredients was prepared in the form of 70% BPC sorbitol solution. The sorbitol was heated to 85°C. and methyl hydroxybenzoate (0.1%) and propyl hydroxybenzoate (0.1%) were added and mixed until dissolved. Citric acid was then added in order to ensure a pH of 5 for the mixture, which was then heated to 95°C. and maintained at this temperature for 15 minutes. The mixture is then allowed to cool to 30°C. and urogastrone added. Urogastrone and cimetidine are added such that each 5 ml. spoonful of the syrup contain 50 µg. of urogastrone and 200 mg. of cimetidine.

# 0161816

- 14 -

## CLAIMS

1.      A pharmaceutical composition comprising (1) human urogastrone or a urogastrone fragment and (2) a known anti-ulcer agent as active ingredients in association with a pharmceutical carrier or excipient.

2.      A composition as claimed in claim 1 in a form adapted for oral administration.

3.      A composition as claimed in claim 1 or claim 2 in dosage unit form.

4.      A composition as claimed in any one of the preceding claims which comprises human urogastrone and a known anti-ulcer agent as active ingredients.

5.      The use of (1) human urogastrone or a urogastrone fragment and (2) a known anti-ulcer agent for the manufacture of a medicament for promoting the healing in mammals of gastrointestinal ulcers.

6.      The use of urogastrone or a urogastrone fragment and a known anti-ulcer agent as claimed in claim 5 wherein the medicament is for promoting the healing of duodenal ulcers.

7.      The use of urogastrone or a urogastrone fragment and a known anti-ulcer agent as claimed in claim 5 or claim 6 wherein the medicament is in a form adapted for oral administration.

8.      The use of urogastrone or a urogastrone fragment and a known anti-ulcer agent as claimed in claim 7 wherein the medicament is in dosage unit form.

9.      For use in promoting the healing of gastrointestinal ulcers in mammals, a pharmaceutical composition adapted for oral administration which comprises as active ingredients (1) human urogastrone or a urogastrone fragment and (2) a known anti-ulcer agent.

MS33079

SD03

JRM/M.TW . 12 Mar 85